Europäisches Patentamt

European Patent Office

Office européen des brevets

(11) Publication number: **0 271 423**
**A1**

# EUROPEAN PATENT APPLICATION

(21) Application number: **87500082.0**

(22) Date of filing: **19.11.87**

(51) Int. Cl.⁴: **A 61 N 1/42**
**A 44 C 5/00**

(30) Priority: **24.11.86 ES 8601353**

(43) Date of publication of application:
**15.06.88 Bulletin 88/24**

(84) Designated Contracting States:
**AT BE CH DE FR GB GR IT LI LU NL SE**

(71) Applicant: **Bocanegra Marquina, Isidro**
**Reyes Catolicos 22**
**E-09005 Burgos (ES)**

(72) Inventor: **Bocanegra Marquina, Isidro**
**Reyes Catolicos 22**
**E-09005 Burgos (ES)**

(74) Representative: **Garcia Cabrerizo, Francisco**
**OFICINA GARCIA CABRERIZO Vitruvio 23**
**E-28006 Madrid (ES)**

(54) **Improved magnetic bracelet.**

(57) An improved magnetic bracelet which is formed by an open metallic ring (1) with two terminals (2) in both ends, the said terminals (2) are made as an independent part and engaged on the bracelet ends, being each body or terminal formed by a permanent magnet (3) housed in shroud (4), like a bushing with its free end closed, while in its opposite end or mouth is engaged a hood-like part (7), which is slidably mounted on the bracelet -and prevented to go out from the latter because the relative end of said bracelet is widened (8); having the magnet a blind port (5) in an inner end in which is housed the end of a rod (6)--emerging from the end area of the bracelet.

Fig. 1

EP 0 271 423 A1

## Description

The invention refers to an improved magnetic bracelet designed to offer to the public in general a medium which has curative properties for some kind of pains or diseases.

At present, there are many types of braceletes named curative, whose purpose is to remove or at least to alleviate some kinds of aches like the rheumatic ones; those caused by the arthrosis; the renal aches or even to improve diseases as those caused by nerves, depression, --stress, etc. The bracelet for those purposes are metallic and they end in spherical terminals, so that these have some given properties that, very often, make the patients that suffer the above-mentioned aching to improve significantly and even to heat entirely.

The bracelet according to the invention is designed -for purposes similar to those just mentioned, although its structure is quite different from all those known at present, since although it is made from metallic material it has its terminals formed by independent parts engaging on the ends of the bracelet, being each terminal formed by a permanent magnet, magnetic or ferrite I, housed in a shroud like a bushing closed by one of its ends, the called outer end, while the opposite end is attached to a rod that emerges partially from the relative end of -the bracelet, holding the magnet and the shroud in which the latter is located by a part fitted in the end are of the bracelet, operating such part like to cover the magnet and to retain the assembly on the relative bracelet end.

The arrangement of the magnet must be so that the poles with the same polarity must be in front each other so that the magnetic flux flows from one pole to another through the bracelet, and it creates a magnetic force in the latter that will have curative properties for the -user, among which the rejuvenation of cells or the healing of the sicks cells in the patients what can result in lots of cures.

The bracelet will be preferably made of stainless --steel not to stain neither the skin of the user, nor --his clothings, being able to be made of another suitable material provided that it has properties and features -adapted to the above-mentioned purposes.

The bracelet is cheap to be produced and has the advantage that the magnets can be replaced as required, -so that its use is unrestricted, unlike the usual braceletes.

To facilitate the understanding of the features of --the invention, is disclosed a detailed description referred to a drawing sheet annexed to this specification and in which as non-limiting example is showed as follows:

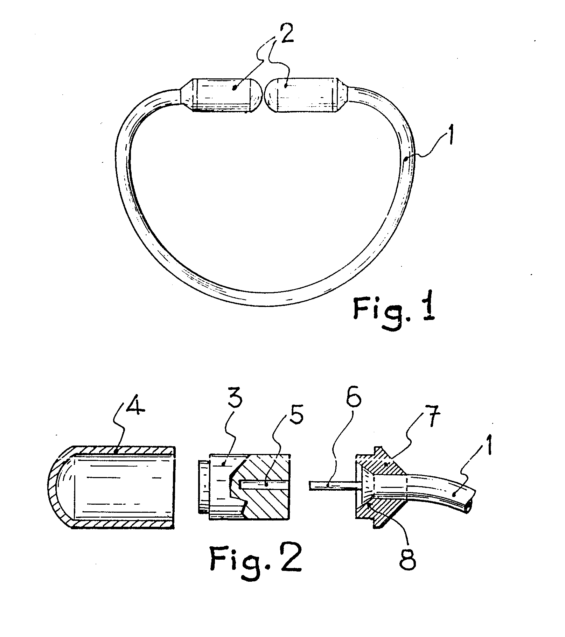

In the fig. 1 is illustrated a general view of the -bracelet according to the invention.

In the fig. 2 is illustrated an exploded section - -view of the parts forming each terminal in both ends of the bracelet.

In said figures, the numerial references are:

1.- Bracelet
2.- Terminals of the bracelet (1)
3.- Magnet fitted in each terminal (2)
4.- Bushing to house the magnet (3)
5.- Blind hole of the end of each magnet (3)
6.- Rod housed in the blind hole (5)
7.- Hood closing each terminal (2)
8.- Widened area of the relative bracelet end (1)

As illustrated in the figures, the magnetic field (1) according to the invention consists of an open metallic ring, preferably of stainless steel, on whose ends are -fitted terminals (2), each constituted by a permanent --magnet (3) located inside a metallic bushing (4) with a closed end that corresponds to the free end and an open end to be able to insert and remove the magnet (3). The outer end of such magnet (3) has a blind port (5) in which houses a portion of a rod (6) which engages from the relative end of the bracelet (1). The magnet (3) remains -hidden inside the bushing (4) through a hood-like part (7) which engages the mouth of the bushing (4), and whose hood is slidably arranged on the bracelet (5) but it can not go out because the relative end of such bracelet has a widened part (8) that acts as an abutment for such hood or cover (7).

The magnets (3) should be fitted so that their positive poles are in front each other, so that the magnetic flux flows through the bracelet (1) between the magnets, causing a magnetization of such bracelet (1) with the above mentioned healing properties.

Although the example refers to a bracelet, it can be appreciated that the invention could be applied to a - -necklace having the same characteristics but a larger --size.

## Claims

1.- An improved magnetic bracelet, designed as a remedy to heat some aching and/or diseases, and being formed by an open metallic ring, preferably of stainless steel, with two terminals in both ends, characterized by the -fact each terminal is made as an independent part and engaged on each end of the bracelet, being each body or --terminal formed by a permanent magnet housed in shroud, like a bushing with its free end closed, while in its --opposite end or mouth is engaged a hood-like part, which is slidably mounted on the bracelet and prevented to go out from the latter because the relative end of said --bracelet is widened; all this so that the part or hood constitutes the retaining means of the magnet and the -coupling and retaining means of the assembly on the end of the bracelet, having the magnet a blind port in an inner end in which is housed the end of a rod emerging from the end area of the bracelet.

2.- An improved magnetic bracelet, according to the claim 1, characterized in that the magnets are arranged so that they have their positive poles remain opposte in front each other by their free ends, so that the --magnetic flux flows through the bracelet from the first magnet to the second one and this causes a magnetization of said bracelet.

0271423

Fig. 1

Fig. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| A | FR-A-1 128 256 (HEMMELER) <br> * Whole document * <br> --- | 1 | A 61 N 1/42 <br> A 44 C 5/00 |
| A | GB-A-2 087 709 (PROMAG) <br> * Whole document * <br> --- | 1 | |
| A | FR-A-2 336 146 (TDK) <br> * Page 2, line 17 - page 3, line 39; figures 4,5 * <br> --- | 1 | |
| A | EP-A-0 035 932 (FLUX YANG) <br> * Page 6, lines 27-34; figure 5 * <br> ----- | 1 | |
| | | | TECHNICAL FIELDS SEARCHED (Int. Cl.4) |
| | | | A 61 N |

The present search report has been drawn up for all claims

| Place of search | Date of completion of the search | Examiner |
|---|---|---|
| THE HAGUE | 29-02-1988 | LEMERCIER D.L.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)